# EUROPEAN PATENT APPLICATION

(11) **EP 4 643 900 A1**
(43) Date of publication of application: **05.11.2025**
(21) Application number: 23911024.0
(22) Date of filing: 18.12.2023
(51) Int. Cl.: A61M 5/14, A61M 39/10, A61M 39/16, A61M 39/18, A61M 5/48

(54) **INTRAVENOUS PERFUSION DEVICE**

(30) Priority: 30.12.2022 ES 202232197 U
(71) Applicant: Universidad de Almería, 04120 la Cañada de San Urbano (ALMERÍA) (ES)
(72) Inventor: GUTIÉRREZ PUERTAS, Lorena, 04120 La Cañada de San Urbano - Almería (ES); MÁRQUEZ HERNÁNDEZ, Verónica V, 04120 La Cañada de San Urbano - Almería (ES); GUTIÉRREZ PUERTAS, Vanesa, 04120 La Cañada de San Urbano - Almería (ES); AGUILERA MANRIQUE, Gabriel, 04120 La Cañada de San Urbano - Almería (ES); LÓPEZ GUTIÉRREZ, Gema, 04120 La Cañada de San Urbano - Almería (ES)
(74) Representative: Pons IP
(86) International application number: PCT/ES2023/070754
(87) International publication number: WO 2024/141687

(57) **Abstract**

The invention relates to an intravenous perfusion device (1) intended to be connected between a connection point (2) of a bag (3) containing a liquid solution (4) and an intravenous catheter (5), wherein the device (1) comprises a drip chamber (7), with a compartment (24) and a punch (8) intended to be inserted into the bag (3) to fill the compartment (24) with the liquid solution (4), an extension set (6) to transport the liquid solution (4), a distal protector (10) with a first extendable pleated tubular body (11), able to be coupled to the compartment (24) and to the connection point (2) of the bag (3), and a proximal protector (14) with a second extendable pleated tubular body (15), able to be coupled to the intravenous catheter (5).

## Description

### OBJECT OF THE INVENTION

The present invention relates to an intravenous perfusion device belonging to the field of medicine, and more specifically to elements used for the intravenous injection of substances into a patient.

The object of the present invention is an intravenous perfusion device, for administering mainly drugs, fluid therapy, blood, blood components, parenteral nutrition or other liquid solutions, especially designed to prevent the spread of microorganisms intravenously and to prevent the development of pressure ulcers in the clinical setting.

### BACKGROUND OF THE INVENTION

Intravenous therapy, which is a method that uses a venous access route to administer liquid solutions to the patient, is common practice in the clinical setting.

Currently, to administer a liquid solution, e.g., serum, medication, blood, blood components, parenteral nutrition, etc., intravenously, a plastic bag or rigid glass bottle containing the liquid solution to be administered and a perfusion system that connects the bag containing the liquid solution to the intravenous catheter carried by the patient are used.

When a liquid solution is administered intravenously, the intravenous perfusion system is connected to the bag containing the liquid solution, leaving the connection between the bag containing the liquid solution and the intravenous perfusion system exposed. The same occurs with the connection of the extension set or tubing that is assembled to the central or peripheral venous catheter carried by the patient. Experience has shown that the connection of the intravenous catheter to the intravenous perfusion system and/or the intravenous perfusion system to the bag containing the liquid solution to be administered can lead to contamination by different types of microorganisms. Currently, in the clinical setting, protection of the distal and proximal connections of the intravenous perfusion system is ensured by placing gauze preferably impregnated with an antiseptic or a transparent film to avoid nosocomial infections. The gauze or transparent film that is placed on the distal and proximal connection of the intravenous perfusion system may become detached, displaced, damaged, or not fully adapted to the connections, thus increasing the risk of contamination, which may lead to nosocomial infection of the patient.

Another drawback of this method is that if any complication occurs during the intravenous infusion of the liquid solution, such as air bubbles entering the intravenous perfusion system, the proximal connection of the intravenous perfusion system must be disconnected in order to purge the air bubbles from the extension set that connects to the intravenous catheter carried by the patient. This involves removing the gauze or transparent film covering the proximal connection, which involves excessive handling of the connection point that joins the intravenous perfusion system to the intravenous catheter carried by the patient. Handling the connection increases the risk of bloodstream infections. Sometimes these infections can lead to the death of the patient. Similarly, this removal of the gauze or transparent film to connect/disconnect the connection of the intravenous perfusion system to the intravenous catheter carried by the patient may increase the risk of leakage of said intravenous line.

On the other hand, research shows that a high percentage of pressure ulcers occurring in the clinical setting are caused by clinical devices, such as intravenous perfusion systems. In particular, in the area where the intravenous perfusion system is assembled with the intravenous catheter. This gauze or transparent film can minimise the pressure exerted by these devices on the patient's skin. However, they are not suitable for these purposes, as there are other types of products that have been shown to prevent the risk of developing pressure ulcers.

Another problem with this method is that healthcare professionals sometimes fail to place the gauze or transparent film on the connections of the intravenous perfusion system. This is mainly caused by healthcare staff forgetting or new staff not knowing that they have to place the gauze or transparent film on the connections of the intravenous perfusion system.

Actually carrying out this method involves additional time, both in collecting the necessary material and in carrying out the method, as the healthcare professional has to connect the intravenous perfusion system to the bag containing the liquid solution to be administered and to the intravenous catheter carried by the patient. The gauze is then impregnated with an antiseptic and placed on the connections of the intravenous system, which is lastly fixed with adhesives or a transparent film. This makes it more complicated to carry out the method when time can be of the essence, especially in an emergency situation. In this sense, reducing the time spent on carrying out this method would allow healthcare professionals to use this time to improve the quality of care provided to patients in the clinical setting.

### DESCRIPTION OF THE INVENTION

The present invention relates to an intravenous perfusion device that solves the problems described above in the prior art, incorporating for this purpose protectors at each of the ends thereof (distal and proximal) that prevent contamination in the intravenous administration of liquid solutions, mainly drugs, fluid therapy, blood, blood components or parenteral nutrition.

The device is intended to be coupled on the one hand to a connection point of a bag containing the liquid solution to be administered, and on the other hand to an intravenous catheter to be applied to the patient, and the protectors are intended to protect the critical connection areas for connecting to the bag and to the catheter.

The device essentially comprises a drip chamber that connects to the bag, a hollow extension set that extends from the drip chamber through which the liquid substance contained in the bag would circulate and ends in a male connector connectable to the intravenous catheter. The device also comprises the two protectors: a distal protector that secures and seals the junction between the drip chamber and the connection point of the bag, and a proximal protector that secures and seals the junction between the male connector and the intravenous catheter.

The drip chamber consists of a filling intake compartment and a hollow piercing punch that is intended to pierce the bag when inserted through the connection point of the bag and allow the liquid solution to pass from the bag into the filling intake compartment.

The distal protector comprises an extendable pleated tubular body having a first upper end arranged around the drip chamber and a first lower end that is intended to embrace the connection point of the bag in a situation of use where the first tubular body is extended, partially covering the drip chamber and the connection point.

The proximal protector comprises a second extendable pleated tubular body having a second upper end arranged around the male connector and a second lower end that is intended to embrace a female connector that the intravenous catheter has, in a situation of use where the second tubular body is extended, thus covering the junction between the male connector and the female connector.

The protectors are linked to the drip chamber and to the male connector, respectively, to ensure that the device always incorporates the protectors.

The distal protector makes it possible to keep the connection between the hollow piercing punch and the connection point of the bag containing the liquid solution to be administered protected, and the proximal protector makes it possible to keep the connection between the male connector and the female connector of the intravenous catheter protected, thus avoiding contamination of the liquid to be injected into the patient.

Both the distal and proximal protectors may incorporate fins at the lower end thereof intended to be grasped by a user to facilitate the extension and retraction movement of the pleated tubular body and thus facilitate handling so that the protectors can be placed more easily at the connection sites.

It is also envisaged that the proximal protector may have two adhesives emerging from the sides of the body, which adhere onto the catheter after removal of the paper protecting the adhesives, to improve the fixation between the perfusion device and the intravenous catheter carried by the patient.

It should also be noted that the device additionally comprises a regulating valve that can be moved along the length of the extension set and is located between the drip chamber and the male connector.

Furthermore, the device may have an accessory incorporated in the extension set that allows it to be coupled to the different intravenous perfusion pumps that currently exist or may exist on the market. In this case, liquid substances that require close monitoring during administration can be administered and the use of the intravenous perfusion device in newborns and the paediatric population is guaranteed.

The protectors may be made of an elastic material, capable of sliding, moulding and coupling tightly to the structures, for example, Lycra and/or Spandex, flexible elastomeric foam or elastomers, with a suitable thickness to prevent the protector from breaking when placed or removed, and capable of adapting to the surface to be covered without deterioration. Furthermore, the protectors may have a treatment composition such as an antimicrobial and/or disinfectant and/or decontaminant. Alternatively, the protectors may be configured so that the pleated tubular body is made of a material, for example, polymer, different from the material of the ends.

In particular, the proximal protector may be made of a multilayer material, such as a three-layer film comprising an inner middle layer made of Lycra and/or Spandex, flexible elastomeric foam or elastomer and an outer layer made of a hypoallergenic, flexible material that is capable of adapting to the shape of the device, such as viscoelastic polyurethane foam, preferably open-pored to promote breathability.

It can be concluded that intravenous administration of liquid solutions, such as drugs, fluid therapy, blood, blood components or parenteral nutrition, is advantageously achieved in a safe manner by means of the device of this invention. Thus, the device simplifies the method of intravenous administration of liquid solutions, reduces handling of the connections, minimises contamination by viruses, parasites, chemical substances or physical agents external to the intravenous perfusion device during the administration of liquid solutions, reduces the risk of nosocomial infection that can lead to death of the patient and prevents the development of pressure ulcers.

Furthermore, it avoids the risk of errors or additional steps, reduces the time it takes to carry out the method and avoids forgetting to place the protections on the connections, constituting an unmistakable sign that the method has been carried out, improving the quality of the care provided to the patient.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows a front view of the device in a situation prior to use.
Figure 2 shows a front view of the distal protector in a situation of use.
Figure 3 shows a front view of the proximal protector in a situation of use.
Figure 4 shows a bottom view of the distal protector.
Figure 5 shows a bottom view of the proximal protector.

### PREFERRED EMBODIMENT OF THE INVENTION

A preferred embodiment of the invention is described below with reference to the attached figures.

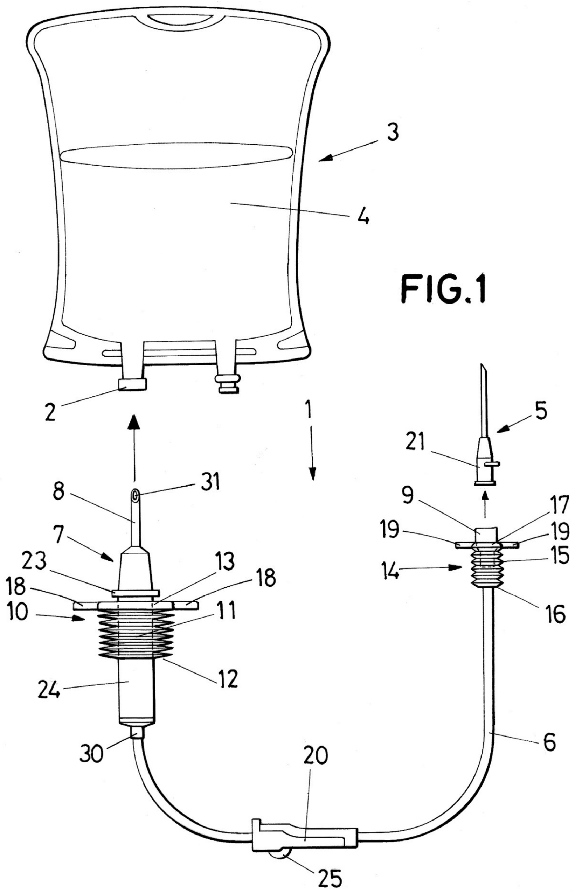

Figure 1 shows a front view of an example of the perfusion device (1) in a situation prior to connection: to the connection point (2) of a bag (3) containing a liquid solution (4), and to the female connection (21) of an intravenous catheter (5).

The device (1) comprises a drip chamber (7), a distal protector (10), an extension set (6), a proximal protector (14) and a male connector (9).

The drip chamber (7) comprises a hollow piercing punch (8) and a filling intake compartment (24). The bevelled punch (8) is intended to be inserted into the connection point (2) of the bag (3) containing the liquid solution (4) to be administered, wherein the punch (8) comprises a longitudinal groove (31) allowing the liquid solution (4) to flow into the compartment (24). This compartment (24) is cylindrical and flexible and is intended to receive the liquid solution (4) to be perfused and allow it to exit through a hole (30) towards the extension set (6).

The extension set (6) allows the liquid solution (4) to flow from the drip chamber (7) towards the male connector (9), where it is envisaged that the device (1) also incorporates a flow regulating valve (20), placed on the extension set (6) and having a wheel (25) that allows the flow of the liquid solution (4) that reaches the male connector (9) to be regulated.

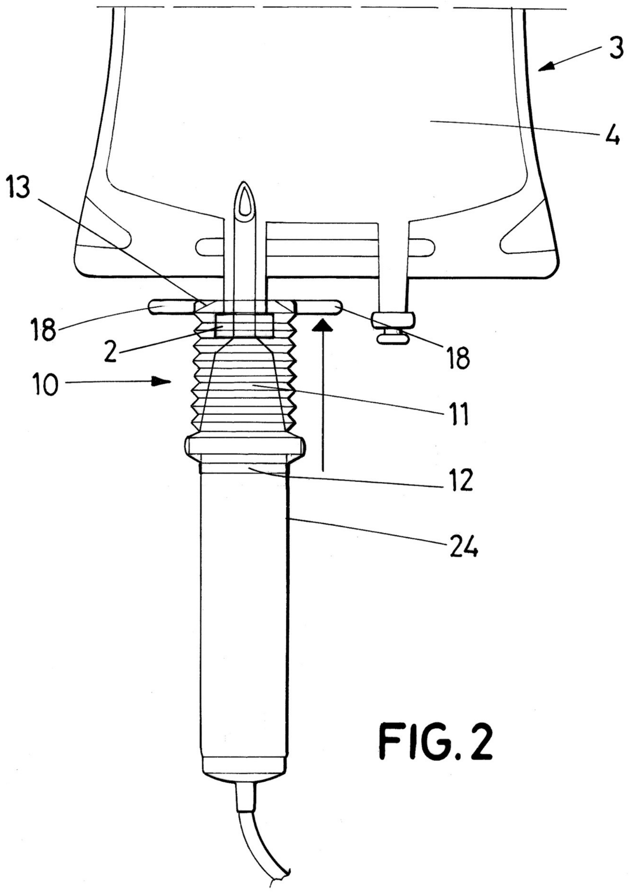

Figure 1 shows the distal protector (10) in its retracted position, comprising a first extendable pleated tubular body (11), which has a first upper end (12) able to be coupled to the drip chamber (7), more specifically to an outer ring (23) of the compartment (24) of the drip chamber (7) and a first lower end (13) able to be coupled to the connection point (2) of the bag (3) in the situation where the pleated tubular body (11) is extended once the punch (8) penetrates the bag (3). Figure 2 shows the distal protector (10) in its extended position where it establishes the described couplings.

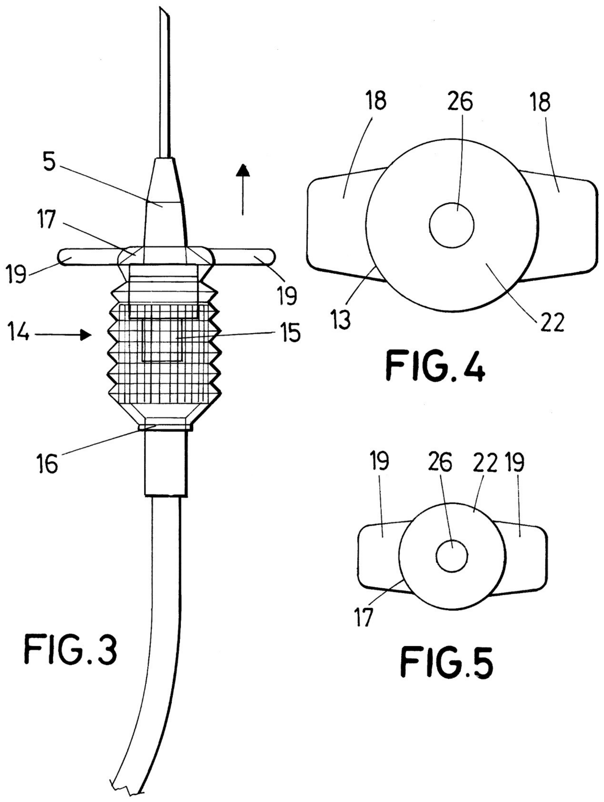

Figure 1 shows the proximal protector (14) in its retracted position, comprising a second extendable pleated tubular body (15), which has a second upper end (16) able to be coupled on the male connector (9) and a second upper end (17) able to be coupled on the female connector (21) of the intravenous catheter (5). Figure 3 shows the proximal protector (14) in its extended position where it establishes the mentioned couplings.

Figures 4 and 5 show that both the first lower end (13) and the second upper end (17) can internally incorporate a flexible annular adapter (22) made of an adaptable material that can contract and expand, such as a rubber band, which has an internal opening (26) to facilitate coupling.

The distal protector (10) is actuated by pulling the first lower end (13) upward until it covers the lower part of the drip chamber (7) and the connection point (2) of the bag (3) being perfectly coupled together, thus ensuring its protection.

In order to facilitate handling of the distal protector (10) in its expansion or retraction movement, the incorporation of first fins (18) that extend perpendicularly from the first lower end (13) is envisaged, as shown in figures 1, 2 or 4. Similarly, the proximal protector (14) also incorporates second fins (19) shown in figures 1, 3 and 5 which extend perpendicularly from the second lower end (17).

Figure 2 shows the situation where the punch (8) has been inserted through the connection point (2) of the bag (3) containing the liquid solution (4) and the distal protector (10) has been coupled. This liquid solution (4) begins to flow down through the punch (8) via a groove (31), in the form of a succession of drops, into the filling intake compartment (24), which is filled with the liquid solution (4).

The liquid solution (4) then passes into the extension set (6) until it reaches the male connector (9) and then the passage of liquid is shut off with the wheel (25) of the flow regulating valve (20).

Once the device (1) has been purged, the male connector (9) is connected to the female connection (21) of the intravenous catheter (5), and the proximal protector (14) is extended and coupled, partially covering the female connection (21) of the intravenous catheter (5).

The device thus connected with the protectors (10, 14) extended and properly coupled is ready to safely carry out perfusion in the patient.

## Claims

1. An intravenous perfusion device (1) for administering liquid substances that is intended to be connected between a connection point (2) of a bag (3) containing a liquid solution (4) and a female connection (21) of an intravenous catheter (5) connectable to a patient, wherein the device (1) comprises:
- a drip chamber (7) having:
∘ a filling intake compartment (24),
∘ a hollow piercing punch (8) extending from the filling intake compartment (24) and intended to be inserted into the bag (3) in a situation of use and allow the liquid solution (4) to pass into the compartment (24),
- a hollow extension set (6) connected at one end to the compartment (24) and intended to transport the liquid solution (4),
- a male connector (9) connected to another end of the extension set (6) intended to be connected to the female connection (21) of the intravenous catheter (5),
**characterised in that** the device (1) further comprises:
a distal protector (10) in turn comprising a first extendable pleated tubular body (11), which has a first upper end (12) able to be coupled to the compartment (24), and a first lower end (13) able to be coupled to the connection point (2) of the bag (3) in the situation where the first pleated tubular body (11) is extended once the punch (8) penetrates the bag (3),
a proximal protector (14) in turn comprising a second extendable pleated tubular body (15), which has a second upper end (16) able to be coupled on the male connector (9) and a second lower end (17) able to be coupled on the female connector (21) of the intravenous catheter (5) in the situation where the second pleated tubular body (15) is extended.

2. The device (1) of claim 1, further comprising an outer ring (23) on the compartment (24) of the drip chamber (7) to which the first upper end (12) of the distal protector (10) is coupled.

3. The device (1) of claim 1, further comprising first fins (18) that extend perpendicularly from the first lower end (13) of the distal protector (10).

4. The device (1) of claim 1, further comprising second fins (19) that extend perpendicularly from the second lower end (17) of the proximal protector (14).

5. The device (1) of claim 1, wherein the first lower end (13) of the distal protector (10) internally incorporates a flexible annular adapter (22) made of an adaptable material that can contract and expand, which has an internal opening (26) to facilitate coupling.

6. The device (1) of claim 1, wherein the second lower end (17) of the proximal protector (14) internally incorporates a flexible annular adapter (22) made of an adaptable material that can contract and expand, which has an internal opening (26) to facilitate coupling.

7. The device (1) of claim 1, wherein the first pleated tubular body (11) is made of a polymer material.

8. The device (1) of claim 1, wherein the second pleated tubular body (15) is made of a polymer material.
